# EUROPÄISCHE PATENTSCHRIFT

(11) **EP 0 837 060 B1**
(45) Veröffentlichungstag und Bekanntmachung des Hinweises auf die Patenterteilung: **04.04.2001**
(21) Anmeldenummer: 97117720.9
(22) Anmeldetag: 11.10.1997
(51) Int. Cl.: C07D 301/02, C07D 303/20, C07F 7/18

(54) **Herstellung eines Epoxids**
Preparation of an epoxide
Préparation d'un époxyde

(30) Priorität: 18.10.1996 EP 96116742
(43) Veröffentlichungstag der Anmeldung: 22.04.1998
(73) Patentinhaber: F. HOFFMANN-LA ROCHE AG, 4070 Basel (CH)
(72) Erfinder: Hilpert, Hans, 4153 Reinach (CH); Widmer, Erich, 4142 Münchenstein (CH)
(74) Vertreter: Kellenberger, Marcus, Dr.

(56) Entgegenhaltungen:
- WO-A-93/23388
- US-A- 5 126 128
- H. MAYER ET AL.: "Synthese von optisch aktiven, natürlichen Carotinoiden und strukturell verwandten Naturprodukten. IV. Synthese von (3R,3'R,6'R)-Lutein" HELVETICA CHIMICA ACTA, Bd. 63, Nr. 6, 17.September 1980, BASEL, CH, Seiten 1451-5, XP002051472
- K.M. SADHU ET AL.: "(Chloromethyl)lithium in an efficient conversion of carbonyl compounds to chlorohydrins or oxiranes" TETRAHEDRON LETTERS, Bd. 27, Nr. 7, 1986, OXFORD, GB, Seiten 795-8, XP002051473
- R. TARHOUNI ET AL.: "Monohalomethyllithium XCH2Li: Stabilization of a potential synthetic reagent" TETRAHEDRON LETTERS, Bd. 25, Nr. 8, 1984, OXFORD, GB, Seiten 835-8, XP002051474

## Beschreibung

Die vorliegende Erfindung betrifft ein Verfahren zur Ueberführung eines geschützten 4-Hydroxy-2,6,6-trimethyl-2-cyclohexen-1-ons (geschützten "Phorenols") in das entsprechende geschützte allylische Epoxid 4,8,8-Trimethyl-1-oxaspiro[2.5]octa-4-en-6-ol unter Verwendung eines zu diesem Zweck noch nie verwendeten Reagens. Sowohl das Ausgangsmaterial als auch das Endprodukt sind bekannte wertvolle Zwischenprodukte zur Herstellung von Carotinoiden.

Die bisher verwendete Methode für die obenerwähnte Ueberführung (eine "C₁-Homologisierung") involvierte als Reagens Dimethylsulfoniummethylid [siehe beispielsweise Pure & Appl. Chem. 51, 535-564 (1979), insbesondere Seiten 546-547: 52 + 53 → 54; sowie Helv. Chim. Acta 63, 1451-1455 (1980), insbesondere Seite 1452: 5 → 6]. Dieses Reagens ist allerdings umständlich herzustellen und liefert als Abfallprodukt das geruchsbelästigende Dimethylsulfid, was schwerwiegende Nachteile darstellen.

Tetr. Lett. 25, No. 8, 835-838 (1984) beschreibt die Herstellung von Monohalogenmethyllithium XCH₂Li durch Brom-Lithium-Austausch aus dem entsprechenden Bromhalogenmethan XCH₂Br und einem Alkyllithium, insbesondere sek. Butyllithium, im Gegenwart von stabilisierendem Lithiumbromid bei tiefen Temperaturen in einem Gemisch von Tetrahydrofuran, Ether und Pentan als Lösungsmittel. Das so hergestellte XCH₂Li eigne sich als Mittel zur C₁-Homologisierung von Aldehyclen oder Ketonen zu den entsprechenden Oxiranen, wobei wenige (fünf) Beispiele dieser Anwendung angegeben sind.

Tetr. Lett. 27, No. 7, 795-798 (1986) nimmt Bezug u.a. auf den obigen Tetr. Lett.-Artikel und offenbart Aehnliches sowie analoge Beispiele der Verwendung von in situ-generiertem Chormethyllithium zur C₁-Homologisierung, und zwar mit und ohne Stabilisierung durch Lithiumbromid. Zudem wird in diesem weiteren Artikel behauptet, dass die Ausbeuten der Oxiran-Herstellung mit in situ-generiertem Chlormethyllithium im allgemeinen grösser sind als mit dem bisher verwendeten Mittel, Dimethylsulfoniummethylid.

Auch die US-Patentschrift 5.126.128 und die PCT-Patentpublikation WO 93/23388 offenbaren die Verwendung eines in situ-generierten Halogenmethyllithiums oder von alternativen Mitteln zur C₁-Homologisierung. Im ersteren Fall wird ein Epoxyketon, und im letzteren Fall ein α-Amino-aldehyd dadurch C₁-homologisiert.

Aufgabe der vorliegenden Erfindung ist es, ein Verfahren zur Herstellung des gegebenenfalls geschützten 4,8,8-Trimethyl-1-oxaspiro[2.5]octa-4-en-6-ols ausgehend von geschütztem Phorenol unter Verwendung eines alternativen Reagens zu entwickeln, und dadurch die obenerwähnten Nachteile bei der Verwendung von Dimethylsulfoniummethylid zu vermeiden. Die Lösung dieser Aufgabe besteht in der Verwendung eines halogenierten Methyllithiums als Reagens in der C₁-Homologisierung. Gegenüber dem bisher verwendeten Reagens bringt die Verwendung des halogenierten Methyllithiums ausserdem Kosteneinsparungen mit sich.

Das erfindungsgemässe Verfahren zur Herstellung des gegebenenfalls geschützten 4,8,8-Trimethyl-1-oxaspiro[2.5]oct-4-en-6-ols der allgemeinen Formel 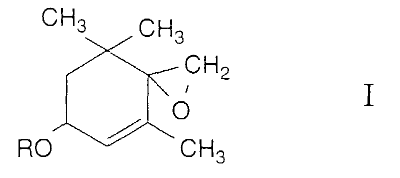 worin R Wasserstoff oder eine Schutzgruppe für Hydroxy bedeutet,
ausgehend von geschütztem Phorenol der allgemeinen Formel 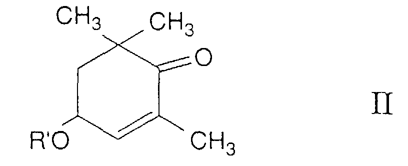 worin R' eine Schutzgruppe für Hydroxy bedeutet,
ist dadurch gekennzeichnet, dass man das geschützte Phorenol mit halogeniertem Methyllithium umsetzt und gewünschtenfalls vom so erhaltenen geschützten 4,8,8-Trimethyl-1-oxaspiro[2.5] oct-4-en-6-ol der Formel I, in der R die Schutzgruppe R' bedeutet, diese Schutzgruppe abspaltet.

Der oben verwendete Ausdruck "Schutzgruppe für Hydroxy" umfasst übliche, besonders auf dem Gebiet der Carotinoide geläufige Schutzgruppen für in der diesbezüglichen strukturellen Umgebung befindliche Hydroxygruppen. Es kommen als diesbezüglich geschützte Hydroxygruppen insbesondere veretherte Hydroxygruppen in Frage. Es handelt sich dabei beispielsweise um C₁₋₅-Alkoxygruppen, vorzugsweise Methoxy und Ethoxy; Mono- und Dialkoxyalkoxygruppen enthaltend bis zu 16 Kohlenstoffatome, vorzugsweise 1-Methoxy-1-methylethoxy [die sogenannte Isopropenylmethylether ("IPM")-Schutzgruppe aufweisendes Hydroxy] bzw. Dimethoxymethoxy; Arylalkoxygruppen, vorzugsweise Benzyloxy; Tetrahydropyranyloxy; und Silyloxygruppen, vorzugsweise Trimethylsilyloxy und Phenyldimethylsilyloxy.

Falls im obigen nicht weiter qualifiziert, umfasst der Ausdruck "Alkyl" geradkettige und verzweigte Gruppen, vorzugsweise mit 1-5 Kohlenstoffatomen, wie beispielsweise Methyl, Ethyl, n-Propyl, Isopropyl, tert.Butyl und dergleichen. Dies gilt auch für den Alkylteil von jedem "Alkoxy". Falls eine Arylgruppe vorkommt, ist diese vorzugsweise Phenyl, welches gegebenenfalls, z.B. mit einer oder mehreren C₁₋₅-Alkyl- und/oder Nitrogruppen, substituiert ist. In der Regel ist jedoch unsubstutiertes Phenyl bevorzugt.

Besonders bevorzugte Schutzgruppen sind die IPM- und die Trimethylsilyl("TMS")-Schutzgruppe.

Die obigen Formeln I und II sind bezüglich der isomeren Form (Konfiguration) neutral dargestellt, umfassen jedoch beliebige isomere (insbesondere optisch isomere) Formen. Gemäss einer Ausführungsform des erfindungsgemässen Verfahrens wird unter Verwendung des halogenierten Methyllithiums geschütztes (S)-Phorenol der Formel II' in geschütztes (3S,6S)-4,8,8-Trimethyl-1-oxaspiro[2.5]oct-4-en-6-ol der Formel I' übergeführt:

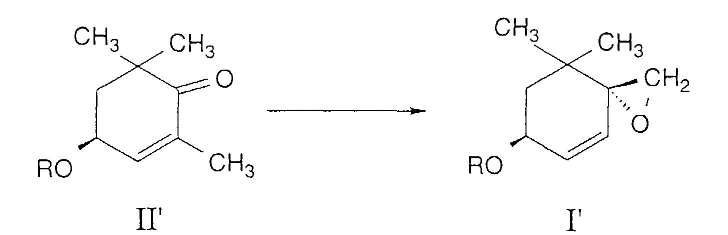

Die C₁-Homologisierung gemäss dem erfindungsgemässen Verfahren erfolgt zweckmässigerweise mit in situ erzeugtem halogeniertem Methyllithium, wobei das geschützte Phorenol der Formel II mit einem halogenierten Methan sowie einem Niederalkyllithium umgesetzt wird. Als Halogen für das dihalogenierte Methan [Hal in CH₂(Hal)₂] eignet sich Fluor, Chlor, Brom oder Jod, wobei die beiden Halogenatome gleich oder verschieden sein können. Hierbei ist Bromchlormethan besonders bevorzugt. Als "Niederalkyl" des Niederalkyllithiums kommt insbesondere geradkettiges oder verzweigtes Alkyl mit bis zu 8 Kohlenstoffatomen in Frage, wie beispielsweise Methyl, Ethyl, n-Propyl, Isopropyl, n-Butyl, Isobutyl, sec.Butyl, tert.Butyl, Pentyl, Hexyl, Heptyl und Octyl. C₁₋₄-Alkyl ist besonders bevorzugt, und ein besonders bevorzugtes Niederalkyllithium ist n-Butyl- oder Hexyllithium. Dieses wird üblicherweise in Lösung in einem niederen Alkan, vorzugsweise Hexan, eingesetzt. Infolge der Umsetzung des dihalogenierten Methans mit dem Niederalkyllithium wird das halogenierte Methyllithium erzeugt, welches daraufhin im selben Reaktionsmedium mit dem geschützten Phorenol reagiert. Beispielsweise wird aus Bromchlormethan und n-Butyllithium das Chlormethyllithium (und n-Butylbromid als Nebenprodukt) erzeugt.

Die C₁-Homologisierung (und die in situ Erzeugung des halogenierten Methyllithiums) wird zudem zweckmässigerweise in einem aliphatischen oder cyclischen Ether als Lösungsmittel bei Temperaturen von etwa -20°C bis etwa -120°C, vorzugsweise von etwa -60°C bis etwa -80°C, durchgeführt. Bei dem Lösungsmittel handelt es sich beispielsweise um Diethylether oder tert.Butyl-methylether bzw. Tetrahydrofuran oder Dioxan. Vorzugsweise wird in Tetrahydrofuran umgesetzt.

Man verwendet pro Aequivalent geschütztes Phorenol zweckmässigerweise etwa 1 bis 2, vorzugsweise etwa 1,1 Aequivalente, des dihalogenierten Methans sowie zweckmässigerweise etwa 1 bis 2, vorzugsweise etwa 1,5 Aequivalente, des Niederalkyllithiums.

Nach Beendigung der Epoxidierung, die normalerweise etwa 30 bis etwa 60 Minuten dauert, wird zur Aufarbeitung das Gemisch zweckmässigerweise aufwärmen gelassen, beispielsweise auf den Temperaturbereich von etwa -70 bis etwa -30°C, mit Wasser oder wässriger Natriumchloridlösung verdünnt und mit einem geeigneten organischen Lösungsmittel, zweckmässigerweise dem für die Umsetzung verwendeten, extrahiert. Nach Abdampfen des Lösungsmittels und eventueller Kristallisation und/oder anderen Reinigungsmethoden erhält man das Produkt der Formel I, worin R die Schutzgruppe R' bedeutet, in guter Reinheit.

Die im erhaltenen Produkt vorhandene Schutzgruppe R' kann gewünschtenfalls nach an sich bekannten Methoden, z.B. durch Hydrolyse mit Säure oder Base, abgespalten werden. Damit wird das 4,8,8-Trimethyl-1-oxaspiro[2.5]oct-4-en-6-ol selber (Verbindung der Formel I, in der R Wasserstoff bedeutet) erhalten.

Das Produkt der Formel I kann nach an sich bekannten Methoden in mehreren Reaktionsstufen in nützliche Endprodukte, z.B. Carotinoide und Pharmazeutika, übergeführt werden [siehe beispielsweise Pure & Appl. Chem. 51, 535-564 (1951)].

Die Erfindung wird durch die nachfolgenden Beispiele veranschaulicht.

### Beispiel 1

### Herstellung von Aceton-methyl[(3S,6S)-4,8,8-trimethyl-1-oxaspiro[2.5]oct-4-en-6-yl]acetal

Eine auf -80°C gekühlte Lösung von 2,00 g (S)-4-(1-Methoxy-1-methylethoxy)-2,6,6-trimethyl-2-cyclohexen-1-on und 1,25 g Bromchlormethan in 20 ml Tetrahydrofuran wurde innert 30 Minuten mit 8,3 ml einer 1,6 molaren Lösung von n-Butyllithium in Hexan versetzt. Das Reaktionsgemisch wurde dann auf -35°C erwärmt und mit 15 ml halbgesättigter Natriumchloridlösung behandelt. Die organische Phase wurde abgetrennt, über wasserfreiem Natriumsulfat getrocknet und unter vermindertem Druck eingeengt. Dies ergab 2,1 g Aceton-methyl[(3S,6S)-4,8,8-trimethyl-1-oxaspiro[2.5]oct-4-en-6-yl]acetal als weissen Festkörper, Smp. 53-59°C; nach Umkristallisation aus Methanol, Smp. 65,2-66,8°C.

### Beispiel 2

### Herstellung von (3R,6S)- und (3S,6S)-Trimethyl-(4,8,8-trimethyl-1-oxaspiro[2.5]octa-4-en-6-yloxy)-silan ausgehend von (4S)-4-Hydroxy-2,6,6-trimethyl-2-cyclohexen-1-on über dessen TMS-geschützte Form (Eintopfverfahren)

Eine auf -80°C gekühlte Lösung von 22,1 g (4S)-4-Hydroxy-2,6,6-trimethyl-2-cyclohexen-1-on in 200 ml Tetrahydrofuran wurde innert 40 Minuten mit 90 ml eine 1,6 molare Lösung von n-Butyllithium in Hexan versetzt und danach innert 10 Minuten mit 20 ml Chlortrimethylsilan behandelt. Anschliessend wurde während 10 Minuten 10,5 ml Bromchlormethan und während einer Stunde 134,5 ml der 1,6 molaren Lösung von n-Butyllithium in Hexan bei -80°C zugetropft, und das Gemisch wurde 20 Minuten gerührt. Das Reaktionsgemisch wurde auf -30°C erwärmt und mit 200 ml halbgesättigter Natriumchloridlösung versetzt. Dann wird die organische Phase abgetrennt, über wasserfreiem Natriumsulfat getrocknet und unter vermindertem Druck eingeengt. Auf diese Weise erhielt man 33,6 g eines dünnschichtchromatographisch reinen Gemisches von (3R,6S) - und (3S,6S)-Trimethyl-(4,8,8-trimethyl-1-oxaspiro[2.5]oct-4-en-6-yloxy)-silan. Massenspektrum: 240/3, M⁺; DC (SiO₂, Hexan/Ethylacetat 1:1): R_{f} = 0,70.

## Patentansprüche

1. Verfahren zur Herstellung eines gegebenenfalls geschützten 4,8,8-Trimethyl-1-oxaspiro[2.5]oct-4-en-6-ols der allgemeinen Formel 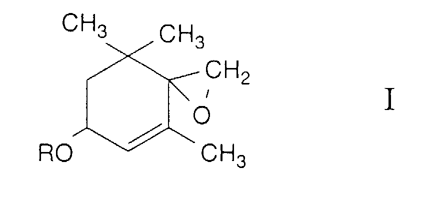 worin R Wasserstoff oder eine Schutzgruppe für Hydroxy bedeutet,
ausgehend von geschütztem 4-Hydroxy-2,6,6-trimethyl-2-cyclohexen-1-on der allgemeinen Formel 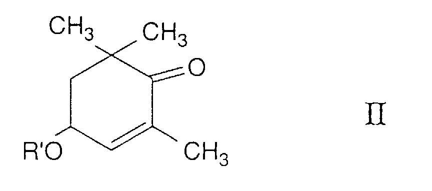 worin R' eine Schutzgruppe für Hydroxy bedeutet,
dadurch gekennzeichnet, dass man das geschützte 4-Hydroxy-2,6,6-trimethyl-2-cyclohexen-1-on mit halogeniertem Methyllithium umsetzt und gewünschtenfalls vom so erhaltenen geschützten 4,8,8-Trimethyl-1-oxaspiro[2.5]oct-4-en-6-ol der Formel I, in der R die Schutzgruppe R' bedeutet, diese Schutzgruppe abspaltet.

2. Verfahren nach Anspruch 1, dadurch gekennzeichnet, dass das halogenierte Methyllithium in situ erzeugt wird, wobei das geschützte 4-Hydroxy-2,6,6-trimethyl-2-cyclohexen-1-on der Formel II mit einem dihalogenierten Methan sowie einem Niederalkyllithium umgesetzt wird.

3. Verfahren nach Anspruch 2, dadurch gekennzeichnet, dass als dihalogeniertes Methan Bromchlormethan eingesetzt wird.

4. Verfahren nach Anspruch 2 oder 3, dadurch gekennzeichnet, dass als Niederalkyllithium n-Butyl- oder Hexyllithium eingesetzt wird.

5. Verfahren nach einem der Ansprüche 1 bis 4, dadurch gekennzeichnet, dass es in einem aliphatischen oder cyclischen Ether als Lösungsmittel durchgeführt wird.

6. Verfahren nach Anspruch 5, dadurch gekennzeichnet, dass als Lösungsmittel Tetrahydrofuran verwendet wird.

7. Verfahren nach einem der Ansprüche 1 bis 6, dadurch gekennzeichnet, dass es bei Temperaturen von etwa -20°C bis etwa -120°C, vorzugsweise von etwa -60°C bis etwa -80°C, durchgeführt wird.

8. Verfahren nach einem der Ansprüche 1 bis 7, dadurch gekennzeichnet, dass die Schutzgruppe (R') für Hydroxy die Isopropenylmethylether- oder Trimethylsilyl-Schutzgruppe ist.

9. Verfahren nach einem der Ansprüche 1 bis 8, dadurch gekennzeichnet, dass geschütztes (S)-4-Hydroxy-2,6,6-trimethyl-2-cyclohexen-1-on in geschütztes (3S,6S)-4,8,8-Trimethyl-1-oxaspiro [2.5]oct-4-en-6-ol übergeführt wird.

## Claims

1. A process for the manufacture of an optionally protected 4,8,8-trimethyl-1-oxaspiro[2.5]oct-4-en-6-ol of the general formula 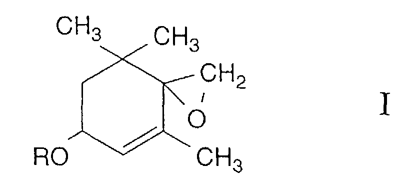 wherein R signifies hydrogen or a protecting group for hydroxy, starting from a protected 4-hydroxy-2,6,6-trimethyl-2-cyclohexen-1-one of the general formula 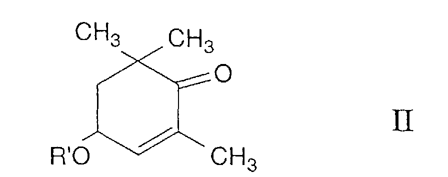 wherein R' signifies a protecting group for hydroxy, which process comprises reacting the protected 4-hydroxy-2,6,6-trimethyl-2-cyclohexen-1-one with halogenated methyllithium and, if desired, cleaving off the protecting group from the thus-obtained protected 4,8,8-trimethyl-1-oxaspiro[2.5]oct-4-en-6-ol of formula I in which R signifies the protecting group R'.

2. A process according to claim 1, wherein the halogenated methyllithium is produced in situ, with the protected 4-hydroxy-2,6,6-trimethyl-2-cyclohexen-1-one of formula II being reacted with a dihalogenated methane and with a lower alkyllithium.

3. A process according to claim 2, wherein bromochloromethane is used as the dihalogenated methane.

4. A process according to claim 2 or 3, wherein n-butyl- or hexyllithium is used as the lower alkyllithium.

5. A process according to any one of claims 1 to 4, wherein the reaction is carried out in an aliphatic or cyclic ether as the solvent.

6. A process according to claim 5, wherein tetrahydrofuran is used as the solvent.

7. A process according to any one of claims 1 to 6, wherein the reaction is carried out at temperatures of about -20°C to about -120°C, preferably of about -60°C to about -80°C.

8. A process according to any one of claims 1 to 7, wherein the protecting group (R') for hydroxy is the isopropenyl methyl ether or trimethylsilyl protecting group.

9. A process according to any one of claims 1 to 8, wherein protected (S)-4-hydroxy-2,6,6-trimethyl-2-cyclohexen-1-one is converted into protected (3S,6S)-4,8,8-trimethyl-1-oxaspiro[2.5] oct-4-en-6-ol.

## Revendications

1. Procédé pour la préparation d'un 4,8,8-triméthyl-1-oxaspiro-[2.5]oct-4-én-6-ol éventuellement protégé de formule générale 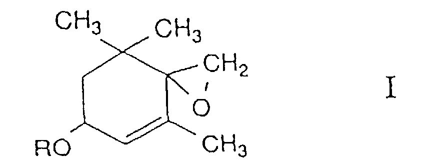 dans laquelle R représente un atome d'hydrogène ou un groupe protecteur de fonction hydroxy, à partir d'une 4-hydroxy-2,6,6-triméthyl-2-cyclohexén-1-one protégée de formule générale 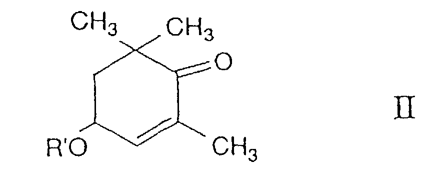 dans laquelle R' représente un groupe protecteur de fonction hydroxy, caractérisé en ce que l'on fait réagir avec un méthyl-lithium halogéné la 4-hydroxy-2,6,6-triméthyl-2-cyclohexén-1-one protégée et, si on le désire, on élimine le groupe protecteur du 4,8,8-triméthyl-1-oxaspiro-[2.5]oct-4-én-6-ol protégé de formule I, ainsi obtenu, dans lequel R représente le groupe protecteur R'.

2. Procédé selon la revendication 1, caractérisé en ce que l'on forme in situ le méthyl-lithium halogéné, en faisant réagir la 4-hydroxy-2,6,6-triméthyl-2-cyclohexén-1-one protégée de formule II avec un méthane dihalogéné ainsi qu'avec un (alkyl inférieur)-lithium.

3. Procédé selon la revendication 2, caractérisé en ce que l'on utilise comme méthane dihalogéné le bromochlorométhane.

4. Procédé selon la revendication 2 ou 3, caractérisé en ce que l'on utilise comme (alkyl inférieur)-lithium le n-butyl-lithium ou l'hexyllithium.

5. Procédé selon l'une des revendications 1 à 4, caractérisé en ce qu'il est effectué dans un éther aliphatique ou cyclique en tant que solvant.

6. Procédé selon la revendication 5, caractérisé en ce que l'on utilise comme solvant le tétrahydrofuranne.

7. Procédé selon l'une des revendications 1 à 6, caractérisé en ce qu'on l'effectue à des températures d'environ -20°C à environ-120°C, de préférence d'environ -60°C à environ -80°C.

8. Procédé selon l'une des revendications 1 à 7, caractérisé en ce que le groupe protecteur (R') de fonction hydroxy est le groupe protecteur éther isopropénylméthylique ou triméthylsilyle.

9. Procédé selon l'une des revendications 1 à 8, caractérisé en ce que la (S)-4-hydroxy-2,6,6-triméthyl-2-cyclohexén-1-one protégée est convertie en (3S,6S)-4,8,8-triméthyl-1-oxaspiro[2.5]oct-4-én-6-ol protégé.
